# EUROPÄISCHE PATENTSCHRIFT

(11) **EP 1 258 264 B1**
(45) Veröffentlichungstag und Bekanntmachung des Hinweises auf die Patenterteilung: **26.12.2007**
(21) Anmeldenummer: 02010211.7
(22) Anmeldetag: 15.05.2002
(51) Int. Cl.: A61M 15/00, A61M 11/00

(54) **Vorrichtung zum Verabreichen von Aerosolen**
Aerosol delivery apparatus
Appareil pour l'administration d'aérosol

(30) Priorität: 16.05.2001 DE 10123749
(43) Veröffentlichungstag der Anmeldung: 20.11.2002
(73) Patentinhaber: Activaero GmbH, 35285 Gemünden (DE)
(72) Erfinder: Scheuch, Gerhard, Dr., 35285 Gemünden (DE)
(74) Vertreter: Vossius & Partner

(56) Entgegenhaltungen:
- EP-A- 0 580 517
- EP-A- 1 136 093
- WO-A-00/58022
- DE-A- 10 013 093
- US-A- 5 156 776
- US-B1- 6 167 880

## Beschreibung

Die Erfindung betrifft eine Vorrichtung zum Verabreichen von Aerosolen und insbesondere eine stationäre Vorrichtung zum kontrollierten Verabreichen therapeutischer Aerosole. Eine stationäre Vorrichtung mit einer Computersteuerung ist z.B. aus WO-A-00/58022 bekannt.

Bei Langstreckenflügen werden heutzutage immer häufiger Personen mit einer akuten Thrombosegefahr befördert und das Risiko, durch einen Langstreckenflug an einer Thrombose zu erkranken, ist nicht zu unterschätzen. Dieses Risiko ist gegeben durch die sitzende Position über einen sehr langen Zeitraum auf beengtem Raum in Kombination mit einer Durchblutungsstörung. Die einzige im Stand der Technik bisher bekannte und auch anerkannte Methode, dieses Risiko zu minimieren, stellt die Injektion eines Heparin-Präparates (in der Regel niedermolekulares Heparin) vor dem Start dar. Da jedoch eine Injektion andere und neue Risiken in sich birgt und nicht nur umständlich, sondern auch sehr unangenehm für die betroffenen Passagiere ist, wird diese Prophylaxe nur in seltenen Fällen angewandt.

Untersuchungen haben ergeben, daß niedermolekulares Heparin grundsätzlich auch per Inhalation verabreicht werden kann. Damit lassen sich prophylaktische Wirkspiegel im Blut erreichen. Die Verabreichung von niedermolekularem Heparin per Inhalation wird jedoch derzeit noch nicht in der Praxis eingesetzt, da die Dosierung per Inhalation nicht gelöst ist, die Dosierung bei diesem Medikament aber ein kritischer Aspekt ist.

Die Dosierung von Medikamenten in Aerosolform in der Inhalationstherapie scheitert bisher hauptsächlich an der Koordination und dem Atemmanöver des Patienten. Darunter ist zu verstehen, wie tief oder flach der Patient inhaliert und wie schnell oder langsam die Atemgeschwindigkeit ist, wie viele Atemzüge der Patient macht und zu welchem Zeitpunkt der Inhalation das Medikament in den Atemzug eingebracht wird. Ein weiterer zu berücksichtigender Punkt sind die Aerosol-physikalischen Eigenschaften des Aerosols, d. h. wie groß das inhalierbare Aerosolteilchen ist, ob es hygroskopische Eigenschaften oder elektrostatische Kräfte gibt etc. Um ein Medikament einer bestimmten Menge präzise dosieren zu können, ist es notwendig, die Abscheidecharakteristika der einzelnen Kompartimente des Atemtraktes zu kennen. Auf der Grundlage dieser Charakteristika sind dann die entsprechenden Parameter auszuwählen, um die Dosierung des Medikamentes in die Lunge zu gewährleisten. Bei dem beschriebenen Anwendungsfall mit Heparin muß der Wirkstoff tief in die Lunge eindringen, damit die Luft/Blutschranke der Alviolen erreicht wird. Nur dann kann Heparin ins Blut gelangen und seine gewünschte Wirkung entfalten.

Sowohl eine Überdosierung als auch eine Unterdosierung eines Wirkstoffes ist problematisch. Eine Unterdosierung ist kritisch, weil im Falle von Heparin keine Thrombose-Prophylaxe erreicht wird und die beschriebenen Gefahren weiter bestehen. Wird der Wirkstoff, wie etwa Heparin, jedoch überdosiert, besteht die Gefahr der inneren und äußeren Blutungen durch die herabgesetzte Gerinnungshemmung des Blutes.

In der Praxis treten bei der Verabreichung von Medikamenten in Aerosolform die folgenden Probleme auf:
1. Viele schwer obstruktive Patienten können einen bestimmten erforderlichen Atemstrom nicht mehr aufbringen, den sie für eine optimale Aerosolapplikation aber erbringen müssten;
2. Viele dieser Patienten können nur noch sehr eingeschränkt Volumina inhalieren, was vor allem bei Patienten mit Lungenemphysem oder Patienten mit sehr kleinen Lungenvolumina der Fall ist; und
3. Jeder Patient inhaliert mit unterschiedlicher Geschwindigkeit und Volumen, so daß es zu starken Schwankungen der in die Lunge gelangenden Medikamentendosis kommt.

Neben der Inhalation von Heparin betrifft die vorliegende Erfindung auch andere Wirkstoffe. Ein ähnlich gelagertes Problem besteht beispielsweise in der Substitutionstherapie von Drogenabhängigen. Auch hier wird bisher die Substitutionsdroge injiziert, obwohl eine Inhalation gleiche, wenn nicht bessere Wirkung hätte und zudem weniger Infektionsrisiken mit sich bringt.

Die EP-A-0 587 380 beschreibt eine Arzneimittelzuführungsvorrichtung, die ein Einatmen erkennt und das Medikament jeweils nur während einer Inhalation appliziert, doch hat der Patient die Freiheit, beliebig zu atmen. Diese Freiheit schwankt überdies von Patient zu Patient, was zu einer erheblichen Variabilität in der Dosierung führt. Was die Praktikabilität dieser Arzneimittelzuführungsvorrichtung anbelangt, so ist diese beispielsweise für die Verabreichung von Heparin als Prophylaxe gegen Thrombose bei beispielsweise Langstreckenflügen ungeeignet, da sie von jedem Patienten mitgeführt werden muß, was sehr lästig ist.

Die Dosierung der inhalierten Aerosole in der Therapie ist bisher nur ungenau möglich und stark von der biologischen Morphometrie und Geometrie abhängig. Außerdem ist diese Dosierung sehr stark vom Patienten individuell aufgebrachten Inhalationsmanöver beeinflußt. Dadurch kann es im schlimmsten Fall dazu kommen, daß in dem Lungenbereich, in dem das Medikament eingebracht werden soll, überhaupt kein Wirkstoff ankommt. Ein weiterer Nachteil ist darin zu sehen, daß bei einem anderen Atemzug - selbst beim selben Patienten - eine Überdosierung des Wirkstoffes erfolgt. Während die physikalischen Aerosoleigenschaften in der Regel gut kontrollierbar und reproduzierbar sind, sind die vom Patienten abhängigen Parameter überhaupt nicht unter Kontrolle.

Ein einfaches Handgerät, mit dem eine Dosis Trockenpulver oder Spray freigesetzt würde hat den Nachteil, daß eine individuelle Dosierung nicht möglich ist. Dies kann nur durch ein aufwendiges individuelles Inhaliersystem ermöglicht werden. Der Patient kann dann aber ebenfalls eine Überdosierung vornehmen, indem zu viele Dosen inhaliert werden, und müßte zudem das Gerät, beispielsweise bei Langstreckenflügen mit sich führen, da es auch für den Rückflug benötigt wird. Die Kosten dafür sind sehr hoch, da jeder Kunde ein eigenes Gerät benötigt.

Demgegenüber liegt der Erfindung die Aufgabe zugrunde, eine verbesserte Vorrichtung zur individuellen kontrollierten Inhalation therapeutischer Aerosole bereitzustellen, die trotz der bereitgestellten Individualität einer großen Anzahl von Nutzern zur Verfügung steht. Diese Aufgabe wird mit den Merkmalen der Ansprüche gelöst.

Die Erfindung stellt eine stationäre Vorrichtung zur individuellen kontrollierten Inhalation therapeutischer Aerosole bereit. Diese stationäre Vorrichtung weist mehrere Wirkstoffspeicher auf, so daß mehrere verschiedene Wirkstoffe vorgehalten und den Benutzern angeboten werden können. Ferner weist die stationäre Vorrichtung eine Wirkstoffausgabeeinrichtung aus. Diese weist vorzugsweise eine Pumpe, eine Dosiereinrichtung und einen Aeorosolierer auf. Weiter vorgesehen ist ein Lesegerät zum Lesen von Speichermedien, auf denen individuelle Patientenparameter und/oder Aerosolparameter für die Inhalation gespeichert sind. Gemäß einer bevorzugten Ausführungsform werden diese für einen bestimmten Patienten individuellen Parameter auf einem Speichermedium erhältlich unter den Bezeichnungen SmartCard, FlashCard oder SmartLabel bereitgestellt. Die individuellen Parameter werden dazu beispielsweise nach einer Messung der aktuellen Lungenfunktion des Patienten (beispielsweise beim Hausarzt) in dem Speichermedium abgelegt. Dieses Speichermedium wird dann vom Patienten mitgeführt und bei Bedarf in die bereitgestellten erfindungsgemäßen stationären Vorrichtungen eingeführt. Außerdem weist die erfindungsgemäße stationäre Vorrichtung eine Steuereinrichtung auf, die mit der Wirkstoffausgabeeinrichtung und dem Lesegerät verbunden ist. Abhängig von den auf dem Speichermedium bereitgestellten individuellen Patienten- und/oder Aerosolparametern steuert die Steuereinrichtung die Wirkstoffausgabeeinrichtung an und gibt eine entsprechende Aerosoldosis aus dem Wirkstoffspeicher an den Patienten frei. Es wird ein erster Fluß (Verneblerfluß) für das Aerosol und gegebenenfalls ein zweiter Fluß (Hilfsfluß) von der der Verneblerluft zugeführten Zusatzluft aufgebaut. Der Patient inhaliert diese Dosis. Da die Abhängigkeit der Deposition von Aerosol in bestimmten Bereichen der Lunge in Abhängigkeit von der Partikelgröße des Wirkstoffes, des Atemvolumens und des Atemflusses bekannt ist, kann somit erfindungsgemäß die Aerosoldeposition in der Lunge im wesentlichen vorherbestimmt und gezielt gesteuert werden. Der Patient empfindet das eingestellte Atemmanöver als angenehm, da es an seine individuellen Möglichkeiten angepaßt ist.

Bevorzugt wird das jeweils vom Patienten aktuell an der Inhalationsvorrichtung durchgeführte Atemmanöver auf dem während des Inhalationsvorganges sich in der Inhalationsvorrichtung befindenden Speichermedium gespeichert, so daß nach einer onsvorrichtung befindenden Speichermedium gespeichert, so daß nach einer gewissen Therapiezeit eine Kontrolle der Anwendung bzw. eine (erneute) Charakterisierung der Lunge durchgeführt werden kann.

Weiter bevorzugt ist das Speichermedium neu programmierbar, um bei Veränderungen der Lungenfunktion des Patienten entsprechend angepaßte Parameter für das richtige Atemmanöver bereitzustellen.

Bevorzugt wird mit der erfindungsgemäßen Inhalationsvorrichtung eine Überdosierung verhindert, beispielsweise durch Vorgeben einer Aktionszeit bzw. einer Aktionssperre, beispielsweise auf dem Speichermedium. Dies verhindert eine erneute Aktivierung der erfindungsgemäßen stationären Inhalationsvorrichtung durch den Patienten, solange nicht die zwischen zwei aufeinanderfolgenden Inhalationen erforderliche Zeit verstrichen ist.

Weiter bevorzugt berücksichtigt die erfindungsgemäße Inhalationsvorrichtung die Pharmakokinetik des verabreichten Wirkstoffes, d.h. die für den Abbau des Wirkstoffes erforderliche Zeit. Heparin beispielsweise wird innerhalb von etwa drei Tagen vollständig abgebaut. Wenn nun mit der erfindungsgemäßen Inhalationsvorrichtung vor einem Flug Heparin inhaliert wird, ein weiterer Flug (Rückflug) schon nach zwei Tagen stattfindet, wäre das Heparin noch nicht vollständig abgebaut und es dürfte vor dem Rückflug lediglich eine geringere Dosis verabreicht werden. Um dies zu erreichen, wird auf dem Speichermedium auch die Pharmakokinetik des Wirkstoffes gespeichert und zusammen mit den anderen Parametern über das Lesegerät eingelesen.

Vorzugsweise dient das Speichermedium auch zu Fehlerprotokollierung. Beispielsweise wird protokolliert, ob eine zu hohe Abweichung des Verneblerdrucks von einem Soll-Bereich aufgetreten ist, oder ob der erforderliche Verneblerdruck überhaupt nicht aufgebaut werden konnte. Ferner wird vorzugsweise eine eventuell bei zu hohem Druck am Mundstück (Beatmungsdruck) auftretende Sicherheitabschaltung auf dem Speichermedium protokolliert. Ferner bevorzugt ist die Protokollierung einer zu hohen Abweichung des Flusses (entweder Verneblerfluß des Aerosols oder Hilfsfluß der der Verneblerluft zugeführten Zusatzluft oder Summe der beiden Flüsse) oder einer Fehlermeldung, wenn einer der genannten Flüsse für die Inhalation nicht aufgebaut werden konnte. Vorzugsweise wird auch ein Abbruch der Inhalation vom Patienten protokolliert.

Die erfindungsgemäße stationäre Vorrichtung zur individuellen kontrollierten Inhalation therapeutischer Aerosole weist die folgenden Vorteile auf:
1. Es kann sehr präzise individuell dosiert werden;
2. Die Therapie ist immer dann zugänglich, wenn sie benötigt wird (beispielsweise Hin-und Rückflug);
3. Ein individuelles Gerät, das mitgeführt werden muß, ist nicht erforderlich;
4. Durch das aufladbare Speichermedium kann eine Medikamenten- und Patienten-Individualisierung erfolgen;
5. Durch das Speichermedium kann eine Mehrfachdosierung verhindert werden;
6. Durch Berücksichtigung der Pharmakokinetik wird eine Überdosierung bei zu kurz aufeinanderfolgenden Inhalationen verhindert.
7.Es können verschiedene Medikamente unterschiedlicher Hersteller in der stationären Vorrichtung vorgehalten und verabreicht werden;
8.Das Atemmanöver kann kontrolliert und die Wirkstoffabgabe an die Deponierung des einzelnen Patienten angepaßt werden; und
9.Die Reproduzierbarkeit der Wirkstoffabgabe wird erhöht.

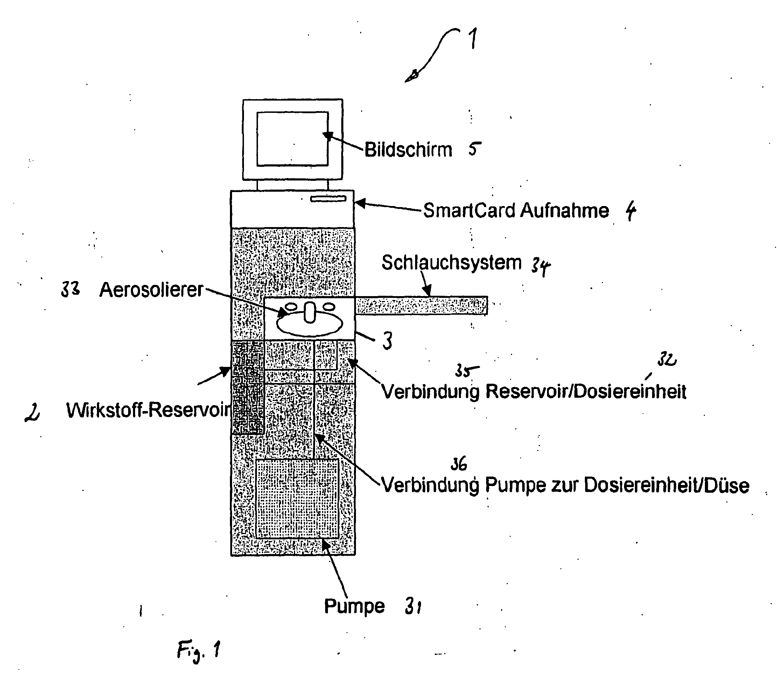

Figur 1 zeigt eine schematische Darstellung der erfindungsgemäßen stationären Vorrichtung gemäß einer bevorzugten Ausführungsform.

Gemäß Figur 1 weist eine bevorzugte Ausführungsform der erfindungsgemäßen Inhalationsvorrichtung 1 ein Wirkstoffreservoir 2 auf. Dieses Wirkstoffreservoir 2 ist mittels einer Verbindung 35 mit einer Wirkstoffausgabeeinrichtung 3 verbunden. Diese Wirkstoffausgabeeinrichtung 3 weist eine Dosiereinheit 32 und einen Aerosolierer 33 zur Aerosolgenerierung auf, und der auszugebende Wirkstoff wird über ein Schlauchsystem 34 an den Patienten ausgegeben. Zur Ausgabe des Wirkstoffes ist eine Pumpe 31 vorgesehen, die über eine Verbindung 36 mit der Dosiereinheit bzw. der Ausgabedüse verbunden ist. Ferner ist ein Lesegerät 4 vorgesehen, mit dem die individuellen Patienten- und/oder Aerosolparameter von einem Speichermedium wie etwa einer SmartCard gelesen werden können. Eine Steuereinrichtung steuert auf der Grundlage der gelesenen Daten die Aerosolausgabe. Letztendlich ist ein Bildschirm 5 vorgesehen, über den der Patient zu entsprechenden Handlungen aufgefordert werden kann.

Erfindungsgemäß inhaliert der Patient über ein Gerät, das stationär an exponierten Orten aufgestellt ist. Für die Heparin-Prophylaxe werden beispielsweise erfindungsgemäße stationäre Inhalationsvorrichtungen in Terminals an Flughäfen aufgestellt. Diese stationären erfindungsgemäßen Inhalationsvorrichtungen, die mit verschiedenen Wirkstoffen befüllt sind, werden mit einem Speichermedium wie etwa einer SmartCard aktiviert. Der Patient hat dazu ein Speichermedium in Form einer Chipkarte, auf der sein individuelles Atemmanöver sowie Daten betreffend das Medikament sowie die Medikamentenmenge, mit dem bei ihm eine optimale Prophylaxe erreicht wird, gespeichert sind. Der Patient führt das Speichermedium in das entsprechende Lesegerät 4 der stationären Inhalationsvorrichtung 1 ein und befestigt beispielsweise ein Einwegmundstück an einem entsprechenden Adapter bzw. an dem Schlauchsystem 34 der Inhalationsvorrichtung 1. Sobald der Patient an diesem Mundstück inhaliert, wird die erfindungsgemäße Inhalationsvorrichtung 1 automatisch in Gang gesetzt und die Steuereinrichtung aktiviert die Wirkstoffabgabe, so dass eine entsprechende Medikamentendosis des für den Patienten geeigneten Medikaments abgegeben wird. Die Inhalationsvorrichtung schaltet automatisch ab, wenn der Patient die benötigte Medikamentenmenge inhaliert hat. Auf dem Speichermedium wird eine weitere Benutzung des gleichen oder eines anderen Terminals gesperrt, um so eine Überdosierung zu verhindern. Der Patient kann somit erst nach einer vorher festgelegten Zeit wieder die für ihn benötigte Medikamentenmenge inhalieren. Ferner wird dem Patient bei Berücksichtigung der Pharamkokonetik lediglich die Menge an Wirkstoff verabreicht, die angesichts der Inhalationshistorie (Abstand zur letzten Inhalation) zulässig ist. Mit Hilfe des Speichermediums/der Karte werden vorzugsweise Kostenabrechnungen abgespeichert, und es wird die Verschreibungspflichtigkeit der Medikation gewährleistet. In der Praxis kann das Speichermedium mit den individuellen Patienten- und/oder Aerosolparametern von einem Hausarzt oder Lungenfacharzt verschrieben werden, der vorher eine entsprechende Lungenfunktionsprüfung durchführt.

Neben der Aufstellung in Flughäfen werden die erfindungsgemäßen stationären Inhalationsvorrichtungen auf anderen leicht zugänglichen Stellen, wie Bahnhöfen oder Vorzimmern von Arztpraxen oder Apotheken aufgestellt, um so beispielsweise eine Substitutionstherapie zu ermöglichen.

Erfindungsgemäß werden auch Medikamente oder Wirkstoffe verabreicht, die eine lang anhaltende atemwegserweiternde Wirkung haben, oder auch Mittel zur Linderung von Erkältungskrankheiten. Dazu ist dann die erfindungsgemäße stationäre Inhalationsvorrichtung mit den entsprechenden Medikamenten ausgestattet.

Die Medikamente befinden sich in entsprechenden Wirkstoffreservoirs 2, welche eine Verbindung 35 zur Dosiereinheit haben (beispielsweise ein Düsenvernebler, Ultraschall-Vernebler oder Trockenpulverdispergierer). Wenn das Speichermedium in das Lesegerät 4 eingeführt wird, wird automatisch die Dosiereinheit entsprechend den auf dem Speichermedium vorgegebenen Parametern gefüllt. Das durchgeführte Atemmanöver des Patienten wird durch die Steuereinrichtung gesteuert, welche auch die Pumpe 31 steuert. Beim ersten Atemzug des Patienten wird die Pumpe 31 oder etwa eine Turbine gestartet, die den notwendigen Druck aufbaut, um die Dosierung des Aerosols und die Atemgeschwindigkeit des Patienten zu steuern. Der Patient kann nur mit der von der erfindungsgemäßen Inhalationsvorrichtung 1 vorgegebenen Geschwindigkeit inhalieren. Nach dem von der Steuereinrichtung berechneten Volumen schaltet das Gerät ab. Der Benutzer atmet daraufhin durch einen Absolutfilter aus, damit die Inhalationsvorrichtung 1 nicht kontaminiert wird, oder der Patient geht von der Inhalationsvorrichtung weg und atmet frei aus. Beim nächsten Atemzug wird wieder das individuelle Atemmanöver getriggert und durchgeführt. Bei Erreichen der optimalen Dosis schaltet die Inhalationsvorrichtung automatisch ab und dem Patienten wird über den Bildschirm 5 angezeigt, dass die Dosierung und Inhalation beendet ist.

Das Einwegmundstück wird entfernt und das Speichermedium aus dem Lesegerät 4 entnommen. Daraufhin kann der Verbindungsschlauch 34 zwischen Mundstück und Inhalationsvorrichtung mit einer Desinfektionslösung gereinigt werden. Dies geschieht durch Aufstecken des Schlauches auf einen entsprechenden Adapter, der mit einer separaten Pumpe verbunden ist, welche das Desinfektionsmittel durch den Schlauch 34 pumpt und welche nach einer bestimmten Zeit den Schlauch auch mit Luftspülung trocknet. Daraufhin ist das Gerät für den nächsten Patienten bereit. Somit können mit einer erfindungsgemäßen stationären Inhalationsvorrichtung mehrere Patienten mit individuellen Inhalationsparametern und unterschiedlich notwendigen Medikamenten bedient werden.

Das Wirkstoffreservoir 2 ist vorzugsweise gekühlt, um dadurch eine mögliche Keimbildung zu verringern. Während der Zufuhr zum Patienten wird die Inhalationsluft aufgewärmt, so daß eine Kondensation bzw. eine Keimbildung im Mundstück verhindert bzw. verringert wird. Alternativ dazu oder zusätzlich ist eine UV-Lichtquelle vorgesehen, die ebenfalls eine mögliche Keimbildung verringert bzw. verhindert. Die entsprechenden Wirkstoffreservoirs sind entweder nachfüllbar oder einfach austauschbar.

Gemäß einer ersten Ausführungsform der erfindungsgemäßen Inhalationsvorrichtung findet die Vernebelung des Aerosols im Inneren der Vorrichtung statt. Durch ein geeignetes Mundstück wird der Wirkstoff während der Inhalation in die Lunge des Patienten gebracht.

Alternativ dazu findet die Vernebelung des Aerosols extern statt und der Vernebler enthält bereits den Wirkstoff. Beispielsweise kann ein Patient einen Vernebler mit Wirkstoff in einer Apotheke (z.B. Flughafenapotheke) beziehen und dann mittels der erfindungsgemäßen Inhalationsvorrichtung den Wirkstoff inhalieren. Einerseits ist diese Ausführungsform hygienischer, da jeder Patient seinen eigenen Vernebler verwendet, andererseits erfolgt bei dieser Ausführungsform die Ausnutzung des Wirkstoffes weniger optimal, da eine Restmenge im Vernebler verbleibt, die nicht mehr genutzt werden kann.

## Patentansprüche

1. Stationäre Inhalationsvorrichtung zur individuellen kontrollierten Inhalation therapeutischer Aerosole mit
einer Wirkstoffausgabeeinrichtung (3);
mehreren mit der Wirkstoffausgabeeinrichtung (3) verbundenen Wirkstoffspeichern (2), in denen unterschiedliche Wirkstoffe vorgehalten werden können;
einem Lesegerät (4) zum Lesen von individuellen Patientenparametem und/oder Aerosolparametern, die auf einem Speichermedium bereitgestellt werden; und
eine Steuereinrichtung, die mit der Wirkstoffausgabeeinrichtung (3) und dem Lesegerät (4) verbunden ist, zum Ausgeben von Wirkstoff abhängig von den gelesenen individuellen Patientenparametern und/oder Aerosolparametern, wobei die Steuereinrichtung so konfiguriert ist, dass sie die gelesenen individuellen Patientenparameter und/oder Aerosolparameter für die Inhalation auswertet und davon abhängig Atemfluss, Atemvolumen und Wirkstoffmenge der Aerosolausgabe der Inhalationsvorrichtung vorgibt.

2. Inhalationsvorrichtung nach Anspruch 1, wobei die Wirkstoffausgabeeinrichtung (3) eine Pumpe (31), eine Dosiereinheit (32) und einen Aerosolierer (33) aufweist.

3. Inhalationsvorrichtung nach Anspruch 2, wobei die Dosiereinheit (32) ein Düsenvernebler, ein Ultraschall-Vernebler oder ein Trockenpulver-Dispergierer ist.

4. Inhalationsvorrichtung nach Anspruch 1, 2 oder 3, wobei das Speichermedium ein FlashCard-, ein SmartCard- oder ein SmartLabel-Speichermedium ist.

5. Inhalationsvorrichtung nach einem der Ansprüche 1 bis 4, wobei das Speichermedium die durchgeführten Atemmanöver speichert.

6. Inhalationsvorrichtung nach einem der Ansprüche 1 bis 5, wobei die Steuereinrichtung für die Einstellung der Wirkstoffmenge ferner die Pharmakokinetik des Wirkstoffes berücksichtigt.

7. Inhalationsvorrichtung nach einem der Ansprüche 1 bis 6, ferner aufweisend Heparin, inbesondere niedermolekulares Heparin, oder eines Wirkstoffes zur Thromboseprophylaxe.

## Claims

1. A stationary inhalation apparatus for an individual controlled inhalation of therapeutic aerosols comprising
a drug-release means (3);
a plurality of drug reservoirs (2) which are connected to the drug-release means (3) and with which different drugs may be applied;
a reader (4) for reading a patient's individual parameters and/or the aerosol parameters stored in a memory means; and
a control unit connected to the drug-release means (3) and the reader (4) for releasing the drug as a function of the read-out individual parameters of the patient and/or the aerosol parameters, wherein the control unit is configured such that it evaluates the read-out individual parameters of a patient and/or the read-out aerosol parameters for the inhalation and controls the respiratory flow, the tidal volume and the drug amount of the aerosol released from the inhalation apparatus as a function thereof.

2. The inhalation apparatus according to claim 1, wherein the drug-release means (3) comprises a pump (31), a dosing means (32) and a disperser (33).

3. The inhalation apparatus according to claim 2, wherein the dosing means (32) is a nozzle atomiser, an ultrasonic atomiser or a dry-powder disperser.

4. The inhalation apparatus according to claim 1, 2 or 3, wherein the memory means is a FlashCard, SmartCard or SmartLabel memory means.

5. The inhalation apparatus according to any of claims 1 to 4, wherein the memory means stores the breathing manoeuvres carried out.

6. The inhalation apparatus according to any of claims 1 to 5, wherein the control unit for controlling the drug amount moreover takes the pharmacokinetics of the drug into account.

7. The inhalation apparatus according to any of claims 1 to 6 comprising heparin, in particular low-molecular heparin, or a drug for preventing thrombosis.

## Revendications

1. Dispositif d'inhalation stationnaire pour l'inhalation contrôlée individuelle d'aérosol thérapeutique avec
un dispositif de délivrance de principe actif (3) ;
plusieurs mémoires de principe actif (2) connectées au dispositif de délivrance de principe actif (3), dans lesquelles peuvent être contenus différents principes actifs ;
un dispositif de lecture (4) pour lire les paramètres de patients individuels et/ou les paramètres d'aérosol qui sont mis à disposition sur un support de mémoire ; et
un dispositif de commande qui est raccordé avec le dispositif de délivrance de principe actif (3) et avec le dispositif de lecture (4) pour la délivrance du principe actif, en fonction des paramètres de patients individuels lus et/ou des paramètres d'aérosol, le dispositif de commande étant configuré de telle sorte que les paramètres de patients individuels lus et/ou les paramètres d'aérosol pour l'inhalation sont évalués et, en fonction de ceux-ci, indiquent le débit respiratoire, le volume respiratoire et la quantité de principe actif de délivrance d'aérosol du dispositif d'inhalation.

2. Dispositif d'inhalation selon la revendication 1, dans lequel le dispositif de délivrance de principe actif (3) présente une pompe (31), une unité de dosage (32) et un système aérosol (33).

3. Dispositif d'inhalation selon la revendication 2, dans lequel l'unité de dosage (32) est un doseur-vaporisateur, un atomiseur ou brumisateur ultrason ou un appareil de dispersion de poudre sèche.

4. Dispositif d'inhalation selon la revendication 1, 2 ou 3, dans lequel le support de mémoire est une carte à puces de type FlashCard ou carte à mémoire instantanée, une SmartCard ou une SmartLabel.

5. Dispositif d'inhalation selon l'une des revendications 1 à 4, dans lequel le support de mémoire mémorise les manoeuvres de respiration effectuées.

6. Dispositif d'inhalation selon l'une des revendications 1 à 5, dans lequel le dispositif de commande tient également compte d'une pharmacocinétique du principe actif pour le dosage de la quantité de principe actif.

7. Dispositif d'inhalation selon l'une des revendications 1 à 6, comprenant de plus de l'héparine, en particulier de l'héparine à faible poids moléculaire, ou un principe actif pour la prophylaxie de la thrombose.
